# EUROPÄISCHE PATENTSCHRIFT

(11) **EP 2 301 485 B1**
(45) Veröffentlichungstag und Bekanntmachung des Hinweises auf die Patenterteilung: **23.09.2015**
(21) Anmeldenummer: 10003996.5
(22) Anmeldetag: 15.04.2010
(51) Int. Cl.: A61F 2/52

(54) **Brustprothese**
Breast prosthesis
Prothèse mammaire

(30) Priorität: 23.09.2009 DE 102009042713
(43) Veröffentlichungstag der Anmeldung: 30.03.2011
(73) Patentinhaber: AMOENA Medizin-Orthopädie-Technik GmbH, 83064 Raubling (DE)
(72) Erfinder: Stelter, Nils, 83112 Frasdorf (DE); Reusch, Michaela, 83395 Freilassing (DE); Leinenbach, Florian, 83059 Kolbermoor (DE); Seehaus, Brigitte, 83128 Halfing (DE)
(74) Vertreter: Laufhütte, Dieter

(56) Entgegenhaltungen:
- DE-U- 7 440 175
- DE-U1- 29 712 015
- DE-U1-202004 003 278
- DE-U1-202005 007 677
- FR-A1- 2 757 043
- US-A1- 2006 025 859

## Beschreibung

Die vorliegende Erfindung betrifft eine Brustprothese mit wenigstens einer Auflagefläche, die für die Auflage auf einer Körperoberfläche bestimmt ist.

Brustprothesen für die Versorgung von Patientinnen nach Brustoperationen, insbesondere nach Brustamputationen sind bereits bekannt. Derartige Brustprothesen werden üblicherweise direkt auf die Körperoberfläche aufgelegt und verweilen dort teilweise über einen längeren Zeitraum, insbesondere über den gesamten Tag.

Durch die direkte Auflage auf der Körperoberfläche können sich Probleme hinsichtlich des Tragekomforts ergeben. Insbesondere ist es möglich, dass es zur Schweißbildung, aber auch Überwärmung des Bereiches der Körperoberfläche kommt, auf dem die Brustprothese aufliegt. Dies wird von den Nutzerinnen als unangenehm empfunden. Darüber hinaus kann es auch zu Reizungen der Haut kommen, was ebenfalls unerwünscht ist.

Ein Vorschlag zur Beseitigung der vorgenannten Probleme besteht darin, die dem Körper zugewandte Seite der Brustprothese als Latentwärmespeicher auszubilden, mittels dem überschüssige Wärme beseitigt werden soll. In entsprechend auf dem Markt befindlichen Brustprothesen ist der Latentwärmespeicher vollflächig ausgeführt.

DE 297 12 015 U offenbart eine Brustprothese mit den Merkmalen aus dem Oberbegriff des Anspruchs 1.

Es ist nun die Aufgabe der vorliegenden Erfindung, eine Brustprothese der eingangs genannten Art in vorteilhafter Weise weiterzubilden, insbesondere dahingehend, dass diese mehr Tragekomfort bietet und Reizungen der Haut vermeidet. Diese Aufgabe wird erfindungsgemäß gelöst durch eine Brustprothese mit den Merkmalen des Anspruchs 1. Danach ist vorgesehen, dass eine Brustprothese wenigstens eine Auflagefläche aufweist, die für die Auflage auf einer Körperoberfläche bestimmt ist, wobei die Auflagefläche mehrere Ausformungen in Form von Noppen aufweist, die sich in unterschiedlichen Höhen aus der Auflagefläche erheben, wobei die Höhe der Noppen zu den Rändern der Auflagefläche hin abnimmt, und wobei mittels der Noppen eine Luftzirkulation und/oder ein Luftaustausch zwischen Auflagefläche und Körperoberfläche erzielbar ist.

Dadurch ergibt sich der Vorteil, dass Luftzirkulation und/oder Luftaustausch zwischen Auflagefläche und Körperoberfläche beim Tragen gewährleistet sind, aber auch, dass die Auflagefläche der Brustprothese auf der Körperoberfläche verringert ist. Dadurch kann der Tragekomfort deutlich erhöht werden. Des Weiteren kann aufgrund der Luftzirkulation und/oder des Luftaustausches eine Überwärmung und damit auch Schweißbildung vermieden werden. Das sich zwischen Auflagefläche und Körperoberfläche ausbildende Luftpolster verbessert zudem das Mikroklima in diesem Bereich.

Mehrere Ausformungen in Form von Noppen, erheben sich in unterschiedlichen Höhen aus der Auflagefläche, wobei die Höhe der Noppen zu den Rändern der Auflagefläche hin abnimmt.

Hierdurch ergibt sich der Vorteil, dass der Übergang an den Rändern der Brustprothese zum Körper der Frau erreicht werden kann. Dies erhöht vorteilhafterweise den Tragekomfort und erzeugt des Weiteren einen optisch ansprechenden Eindruck.

Vorzugsweise ist bzw. sind die Noppe bzw. die Noppen elliptisch und/oder rund und/oder tränenförmig ausgebildet.

Eine Ausbildung der Noppen kann beispielsweise dadurch erfolgen, dass im Herstellungsprozess eine Noppenhöhlung bzw. -form mit oder ohne Vakuum zur Ausformung des Prothesenrohlings vorgesehen ist. Denkbar ist des Weiteren, eine gesonderte Form, etwa eine Noppenschale vorzusehen, die auf bereits verwendete Höhlungen bzw. Formen aufgesetzt werden kann (Cosmetic Layer-Membranhöhlung/Schablone). Denkbar ist, 3D-Spritzguss für die Herstellung zu verwenden oder die Noppen-Formteile einzeln aufzubringen.

Denkbar ist ebenso, dass die Noppen nachträglich auf einem Standardrohling für eine Brustprothese aufgebracht werden können, etwa durch Raupendosierung. Weiter ist möglich, die Noppen aufzuschäumen, etwa durch Integralschaum und/oder thermoplastischen Schaum.

Auch ist vorstellbar, DU Expancel Paste auf den Standardrohling aufzudrucken und thermisch zu aktivieren, etwa nach Art den T-Shirt-Drucks, bzw. DU Expancel in der Silikonrückseite anzuordnen und partiell thermisch zu aktivieren. Die thermische Aktivierung kann durch Infrarot, Hochfrequenz, Heizschablonen oder Laser erreicht werden.

Eine weitere Möglichkeit besteht darin, die Noppen aus einer harten Rückseite auszufräsen, also etwa eine harte Silikonplatte für die Auflagefläche vorzusehen, aus der die Noppen und die Auflagefläche ausgeformt, insbesondere ausgefräst werden.

Es kann vorgesehen sein, dass die Größe der Noppen zumindest zu einem Rand, vorzugsweise jeweils zu allen Rändern, abnimmt und vorzugsweise im mittleren Bereich der Auflagefläche am größten ist.

Möglich ist des Weiteren, dass mehrere Noppen vorhanden sind, die derart auf der Auflagefläche angeordnet sind, dass durch die Anordnung im aufgesetzten Zustand auf der Körperoberfläche wenigstens ein Luftkanal ausgebildet sind, mittels dessen Luft zu- und abführbar ist.

Darüber hinaus kann vorgesehen sein, dass durch den Luftkanal durch Kaminwirkung und/oder durch Pumpdruck bzw. Eindrücken wenigstens eines Teiles der Brustprothese Luft zu- und abführbar ist. Dadurch ergibt sich der Vorteil, Luftzirkulation und/oder Luftaustausch aktiv unterstützen zu können.

Außerdem ist denkbar, dass die Brustprothese wenigstens einen Latentwärmespeicher aufweist oder zumindest teilweise aus Latentwärmespeicher-Material besteht, wobei der Latentwärmespeicher oder das Latentwärmespeicher-Material vorzugsweise PCM-Material ist. Vorzugsweise wird als Latentwärmespeicher oder als Latentwärmespeicher-Material Material verwendet, dessen Schmelzpunkt im Bereich der Körpertemperatur liegt, so dass überschüssige Wärmeenergie durch den stattfindenden Phasenübergang von der festen in die flüssige Phase des Latentwärmespeichers bzw. des Latentwärmespeicher-Materials absorbiert wird.

Des Weiteren ist möglich, dass der Latentwärmespeicher oder das Latentwärmespeicher-Material im Bereich der Auflagefläche angeordnet ist.

Vorteilhafterweise kann vorgesehen sein, dass der Latentwärmespeicher oder das Latentwärmespeicher-Material im Bereich des Mittels angeordnet ist. Insbesondere kann durch die Noppenstruktur in Kombination mit einem PCM-Material eine Optimierung der Wirkung des PCM-Materials erreicht werden, indem Bereiche mit Hautkontakt schnell reagieren, weitere Bereiche in der Noppenmitte durch Wärmeleitung später einsetzen und die Bereiche am Noppenansatz über Luftzirkulation länger anhaltend reagieren. Eine Pufferwirkung kann so vorteilhafterweise erreicht werden. Darüber hinaus kann durch die vergrößerte Oberfläche die Wirkung des PCM-Materials stärker entfaltet werden bzw. besser genutzt werden. Ein deutlich verbesserter Tragekomfort, eine Verhinderung der Überwärmung und eine Verbesserung des Mikroklimas ist vorteilhaft erzielbar.

Darüber hinaus ist denkbar, dass die Noppen, zumindest teilweise durch den Latentwärmespeicher oder das Latentwärmespeicher-Material ausgebildet ist/sind und/oder zumindest teilweise den Latentwärmespeicher oder das Latentwärmespeicher-Material umfasst bzw. umfassen.

Möglich ist ferner, dass die Brustprothese zumindest teilweise aus einer additionsvernetzenden Zwei-Komponenten-Silikon-Kautschuk-Masse besteht oder eine additionsvernetzende Zwei-Komponenten-Silikon-Kautschuk-Masse umfasst.

Weiter kann vorgesehen sein, dass die Brustprothese aus wenigstens einer in Kunststofffolie eingeschweißten Masse ausgebildet und/oder der Brustform nachgebildet ist, wobei die Masse vorzugsweise zumindest teilweise aus einer additionsvernetzenden Zweikomponenten-Silikonkautschuk-Masse besteht und/oder dass die Brustprothese aus einem schalenförmigen Körper aus einem weich-elastisch eingestellten Kunststoff, vorzugsweise einer additionsvernetzten Zwei-Komponenten-Silikon-Kautschuk-Masse, der in Kunststofffolien eingeschweißt ist und so eine erste Kammer bildet, und einer der Trägerin während des Tragens zugewandten und sich an die erste Kammer anschließenden mit einem Füllmaterial füllbaren zweiten Kammer besteht. Als Kunststofffolien können beispielsweise Polyurethan-Folien eingesetzt werden.

Außerdem ist denkbar, dass die Ausformung durch eine in Kunststofffolie eingeschweißte Masse ausgebildet ist, wobei die Masse vorzugsweise zumindest teilweise aus einer additionsvernetzenden Zwei-Komponenten-Silikon-Kautschuk-Masse besteht.

Des Weiteren kann vorgesehen sein, dass die Brustprothese und/oder die Noppen wenigstens teilweise aus Schaumstoff ausgebildet ist bzw. sind. Beispielsweise ist denkbar, dass die Noppen aus Schaumstoff ausgeführt sind. Möglich ist auch, dass sich aus der Auflagefläche Schaumstoffwulste erheben.

Weitere Einzelheiten und Vorteile der Erfindung sollen nun anhand eines in der Zeichnung dargestellten Ausführungsbeispiels näher erläutert werden.

Es zeigen:
- Figur 1:: eine Schnittdarstellung der Brustprothese in einer ersten Ausführungsform in Seitenansicht;
- Figur 2:: eine Rückansicht der Brustprothese in einer weiteren Ausführungsform;
- Figur 3:: eine Rückansicht der Brustprothese in einer weiteren Ausführungsform; und
- Figur 4:: eine Rückansicht der Brustprothese in einer weiteren nicht beanspruchten Ausführungsform.

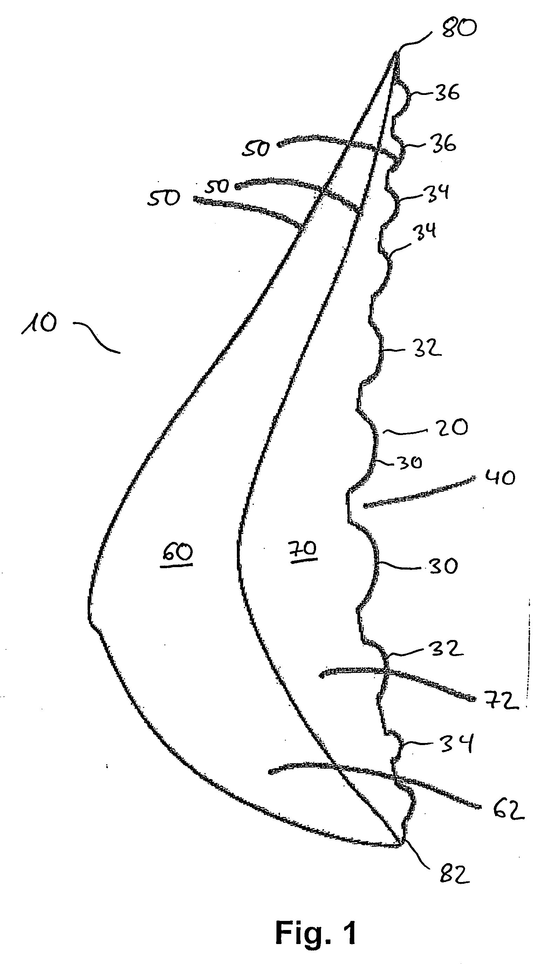

Figur 1 zeigt eine Schnittdarstellung der Brustprothese 10 in einer ersten Ausführungsform in Seitenansicht. Die Brustprothese 10 ist dabei der Form einer weiblichen Brust nachempfunden und kann als rechte oder linke Brustprothese ausgeführt sein. Genausogut ist denkbar, dass eine einzige Ausführungsform der Brustprothese 10 für die rechte oder linke Seite vorgesehen ist. Die Brustprothese 10 ist dabei in der Frontansicht dreiecksförmig und weist konvexe Außenkanten bzw. Ränder 80, 82, 84 auf.

Die Brustprothese 10 weist zwei Kammern 60 und 70 auf, wobei die erste Kammer 60 außenliegend bzw. von der Auflagefläche 20 abgewandt und durch zwei Folien 50 ausgebildet bzw. begrenzt ist. Die Folien 50 sind Polyurethan-Folien 50. Die Kammer 60 mit einer additionsvernetzten Zwei-Komponenten-Silikon-Kautschuk-Masse 62 gefüllt.

Die zweite Kammer 70 ist innenliegend, d. h. eine Außenfläche 20 der Kammer 70 ist eine Auflagefläche 20, die für die Auflage auf die Körperoberfläche vorgesehen ist. Die Kammer 70 ist ebenfalls durch zwei Folien 50 begrenzt, wobei die im Inneren der Brustprothese 10 liegende Folie 50 die Kammern 60 und 70 voneinander trennt. Die Kammer 70 ist dabei vorzugsweise mit Latentwärmespeicher-Material 72, insbesondere PCM-Material 72 gefüllt.

Aus der Auflagefläche 20 erheben sich die Mittel 30, 32, 34, 36, mittels derer eine Luftzirkulation und ein Luftaustausch zwischen der Auflagefläche 20 und der Körperoberfläche (nicht in Figur 1 dargestellt) erzielbar ist. Die Mittel 30, 32, 34, 36 sind dabei als Noppen 30, 32, 34, 36 ausgeführt, die sich aus der Auflagefläche 20 erheben, so dass zwischen den Noppen 30, 32, 34, 36 Luftführungskanäle 40 ausgebildet sind, die eine Luftzirkulation zwischen Auflagefläche 20 und Körperoberfläche ermöglichen.

Die Noppen 30, 32, 34, 36 sind dabei durch die Formgebung der Kammer 70 ausgebildet, etwa durch die Formgebung des von der Folie 50 umgebenen Latentwärmespeichermaterials 72. Genausogut kann jedoch ebenso vorgesehen sein, dass die Noppen 30, 32, 34, 36 auf die Auflagefläche 20 bzw. die Folie 50 gesondert aufgesetzt sind.

Die Höhe und auch die Größe der Noppen 30, 32, 34, 36 nimmt jeweils zu den Rändern 80, 82, also zum oberen Rand 80 und dem unteren Rand 82 der Auflagefläche 20 ab, vorzugsweise auch zu den nicht in Figur 1 dargestellten Seitenrändern 84 der Auflagefläche 20. Die Noppen 30, 32, 34, 36 sind vorzugsweise kreisrund bezogen auf den Querschnitt ausgebildet und erheben sich halbkugelförmig aus der Auflagefläche 20.

Die Noppen 30 in der ersten und größten Höhe und Größe sind dabei im Zentrum der Auflagefläche 20 angeordnet und von den Noppen 32 in der zweiten und nächstkleineren Größe und Höhe umgeben. Daraufhin folgen die Noppen 34 und 36 in der dritten und vierten, jeweils in Höhe und Größe abnehmend. Dadurch ergibt sich der Vorteil, dass sich die Brustprothese an den Rändern 80, 82 besser an den Körper der Frau anlegen kann, was neben einem angenehmen Tragegefühl des Weiteren eine verbesserte optische Wirkung hat.

Die Luftzirkulation und der Luftaustausch zwischen Auflagefläche 20 der Brustprothese 10 und der nicht gezeigten Körperoberfläche erfolgt ohne weiteres Zutun bereits durch den Kamineffekt, so dass die warme Luft abzieht und Umgebungsluft einströmt. Dadurch wird das Mikroklima deutlich zu bisherigen Ansätzen verbessert.

Alternativ oder zusätzlich kann vorgesehen sein, dass durch Eindrücken der Brustprothese 10 die Luftzirkulation und der Luftaustausch zwischen Auflagefläche 20 der Brustprothese 10 und der nicht gezeigten Körperoberfläche aktiv unterstützt wird, da hierdurch die zwischen Auflagefläche 20 der Brustprothese 10 und Körperoberfläche befindliche Luft ausgepumpt und Umgebungsluft in die Zwischenräume 40 bzw. Luftkanäle 40 eingesaugt wird.

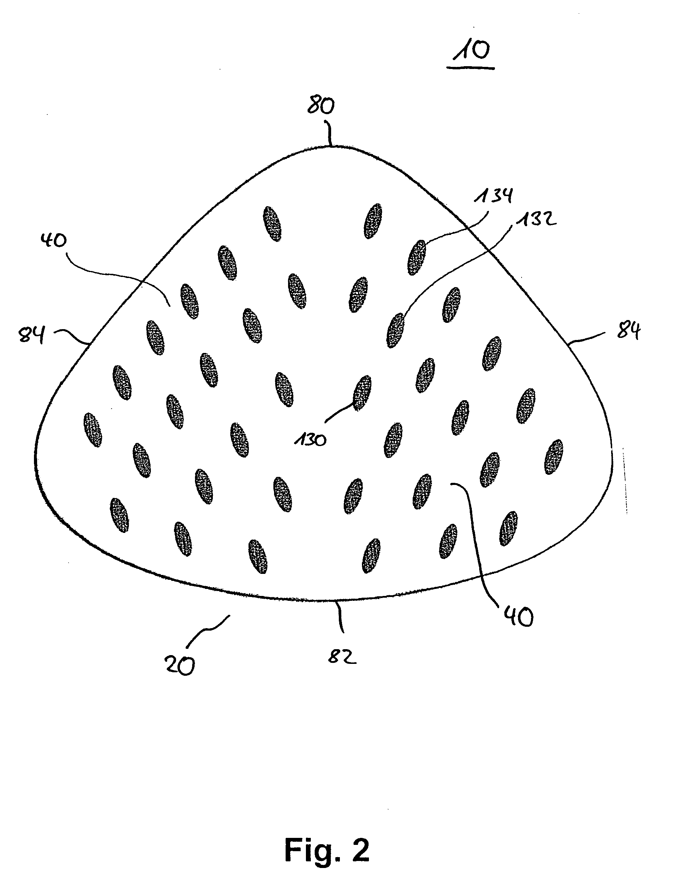

Figur 2 zeigt eine Rückansicht einer weiteren Ausführungsform der Brustprothese 10 mit Blick auf die Auflagefläche 20. Aus der Auflagefläche 20 erheben sich mehrere ovale bzw. elliptische Noppen 130, 132, 134, die sich in unterschiedlichen Höhen aus der Auflagefläche 20 erheben und zwischen denen Zwischenräume 40 vorhanden sind, die zur Luftzirkulation und zum Luftaustausch dienen. Dabei nimmt die Höhe der Noppen 130, 132, 134 jeweils zu den Rändern 80, 82, 84, also zum oberen Rand 80 und dem unteren Rand 82 sowie zu den Seitenrändern 84 der Auflagefläche 20 ab.

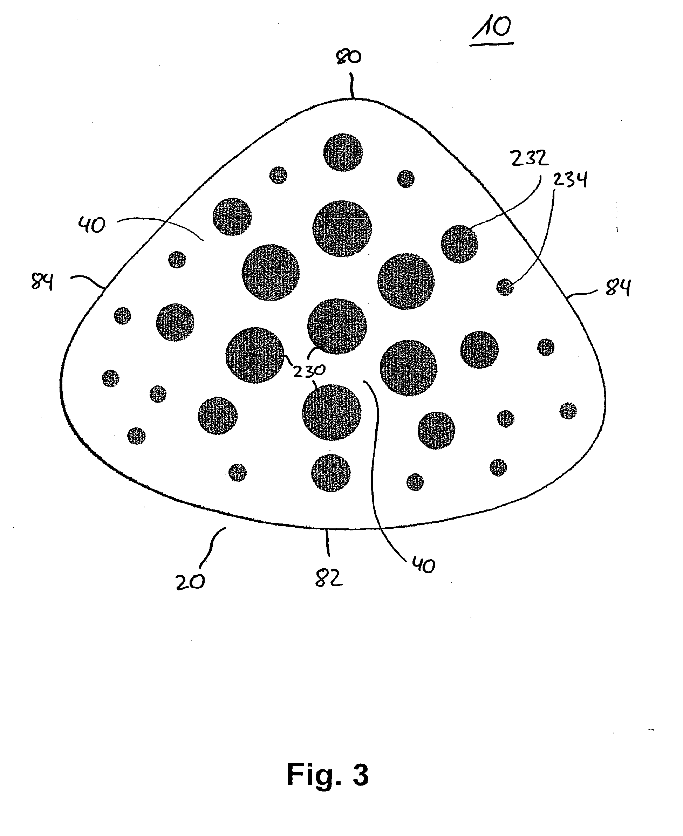

Figur 3 zeigt eine Rückansicht einer weiteren Ausführungsform der Brustprothese 10 mit Blick auf die Auflagefläche 20. Aus der Auflagefläche 20 erheben sich mehrere kreisrunde 230, 232, 234, die in mehreren Höhen und Größen sich aus der Auflagefläche 20 annähernd halbkugelförmig erheben und zwischen denen Zwischenräume 40 vorhanden sind, die zur Luftzirkulation und zum Luftaustausch dienen. Dabei nimmt die Höhe und Größe der Noppen 230, 232, 234 jeweils zu den Rändern 80, 82, 84, also zum oberen Rand 80 und dem unteren Rand 82 sowie zu den Seitenrändern 84 der Auflagefläche 20 ab.

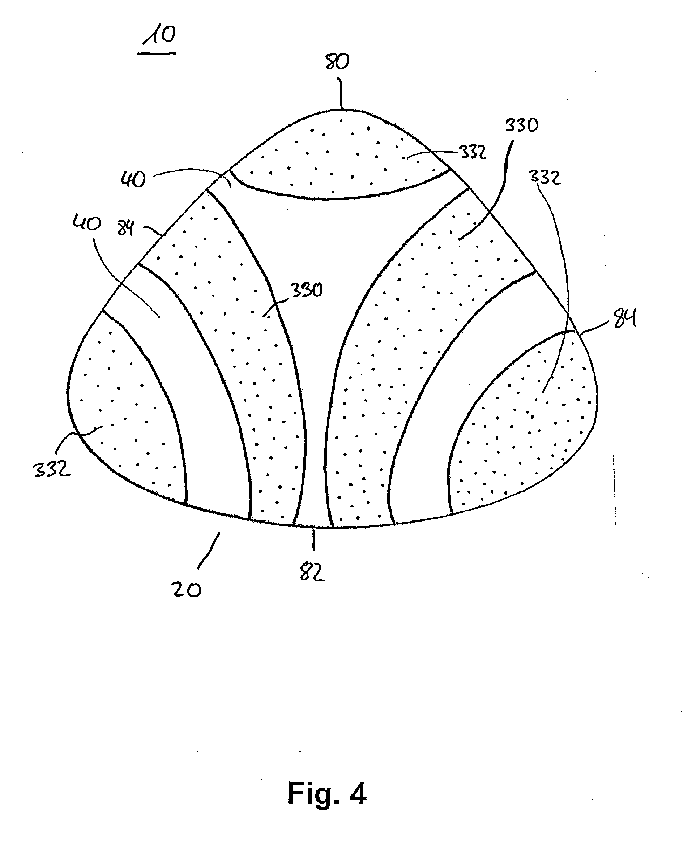

Figur 4 zeigt eine Rückansicht einer weiteren Ausführungsform der Brustprothese 10 mit Blick auf die Auflagefläche 20, wobei die Mittel 330, 332 zur Erzielung eines Luftaustausches zwischen Auflagefläche 20 und Körperoberfläche als Schaumstoff-Ausformungen 330, 332 ausgebildet sind. Die Ausformungen 330, 332 erheben sich dabei aus der Auflagefläche 20, wobei die an den Seitenrändern 80, 82, 84 bzw. in den Eckbereichen liegenden Ausformungen 332 eine geringere Höhe haben als die Ausformungen 330, die zentral in der Auflagefläche 20 angeordnet sind. Die wulstartigen Ausformungen 330 nehmen jedoch ebenfalls in den benachbart zu den Rändern 82, 82 liegenden Bereichen in der Höhe ab. Zwischen den Ausformungen 330, 332 befinden sich Zwischenräume 40 bzw. Luftkanäle 40, durch die ein Luftaustausch ermöglicht wird. Zwischen den beiden Ausformungen 330 und der in der oberen Ecke 80 befindlichen Ausformung 332 ist ein annähernd dreiecksfömigen Zwischenraum 40 ausgebildet, der konkave Seiten aufweist.

## Patentansprüche

1. Brustprothese (10) mit wenigstens einer Auflagefläche (20), die für die Auflage auf einer Körperoberfläche bestimmt ist,
wobei die Auflagefläche (20) mehrere Ausformungen in Form von Noppen aufweist, **dadurch gekennzeichnet, dass** sich die Noppen in unterschiedlichen Höhen aus der Auflagefläche erheben,
wobei die Höhe der Noppen zu den Rändern der Auflagefläche hin abnimmt, und wobei mittels der Noppen eine Luftzirkulation und/oder ein Luftaustausch zwischen Auflagefläche (20) und Körperoberfläche erzielbar ist.

2. Brustprothese (10) nach Anspruch 1, **dadurch gekennzeichnet, dass** die Noppen elliptisch und/oder rund und/oder tränenförmig ausgebildet sind.

3. Brustprothese (10) nach einem der vorhergehenden Ansprüche, **dadurch gekennzeichnet, dass** auch die Größe der Noppen (30, 32, 34, 36, 130, 132, 134, 230, 232, 234) zumindest zu einem Rand (80, 82, 84) und vorzugsweise jeweils zu allen Rändern (80, 82, 84) hin abnimmt, und vorzugsweise im mittleren Bereich der Auflagefläche (20) am größten ist.

4. Brustprothese (10) nach einem der vorhergehenden Ansprüche, **dadurch gekennzeichnet, dass** durch die Noppen im aufgesetzten Zustand der Brustprothese auf der Körperoberfläche wenigstens ein Luftkanal (40) ausgebildet ist, mittels dessen Luft zu- und abführbar ist.

5. Brustprothese (10) nach Anspruch 4, **dadurch gekennzeichnet, dass** durch den Luftkanal (40) durch Kaminwirkung und/oder durch Pumpdruck bzw. Eindrücken wenigstens eines Teiles der Brustprothese (10) Luft zu- und abführbar ist.

6. Brustprothese (10) nach einem der vorhergehenden Ansprüche, **dadurch gekennzeichnet, dass** die Brustprothese (10) wenigstens einen Latentwärmespeicher (72) aufweist oder zumindest teilweise aus Latentwärmespeicher-Material (72) besteht, wobei der Latentwärmespeicher (72) oder das Latentwärmespeicher-Material (72) vorzugsweise PCM-Material ist.

7. Brustprothese (10) nach Anspruch 6, **dadurch gekennzeichnet, dass** der Latentwärmespeicher (72) oder das Latentwärmespeicher-Material (72) im Bereich der Auflagefläche (20) angeordnet ist.

8. Brustprothese (10) nach Anspruch 6 oder 7, **dadurch gekennzeichnet, dass** der Latentwärmespeicher (72) oder das Latentwärmespeicher-Material (72) im Bereich der Noppen angeordnet ist.

9. Brustprothese (10) nach einem der Ansprüche 6 bis 8, **dadurch gekennzeichnet, dass** die Noppen (30, 32, 34, 36, 130, 132, 134, 230, 232, 234), zumindest teilweise durch den Latentwärmespeicher (72) oder das Latentwärmespeicher-Material (72) ausgebildet sind und/oder zumindest teilweise den Latentwärmespeicher (72) oder das Latentwärmespeicher-Material (72) umfassen.

10. Brustprothese (10) nach einem der vorhergehenden Ansprüche, **dadurch gekennzeichnet, dass** die Brustprothese (10) zumindest teilweise aus einer additionsvernetzenden Zwei-Komponenten-Silikon-Kautschuk-Masse (62) besteht oder eine additionsvernetzende Zwei-Komponenten-Silikon-Kautschuk-Masse (62) umfasst.

11. Brustprothese (10) nach einem der vorhergehenden Ansprüche, **dadurch gekennzeichnet, dass** die Brustprothese (10) aus wenigstens einer in Kunststofffolie (50) eingeschweißten Masse (62, 72) ausgebildet und/oder der Brustform nachgebildet ist, wobei die Masse (62, 72) vorzugsweise zumindest teilweise aus einer additionsvernetzenden Zweikomponenten-Silikonkautschuk-Masse (62) besteht und/oder dass die Brustprothese (10) aus einem schalenförmigen Körper aus einem weich-elastisch eingestellten Kunststoff, vorzugsweise einer additionsvernetzten Zwei-Komponenten-Silikon-Kautschuk-Masse (62), der in Kunststofffolien (50) eingeschweißt ist und so eine erste Kammer (60) bildet, und einer der Trägerin während des Tragens zugewandten und sich an die erste Kammer (60) anschließenden mit einem Füllmaterial (72) füllbaren zweiten Kammer (70) besteht.

12. Brustprothese (10) nach Anspruch 11, **dadurch gekennzeichnet, dass** die Noppen durch die in Kunststofffolie (50) eingeschweißte Masse (62, 72) ausgebildet sind, wobei die Masse (62, 72) vorzugsweise zumindest teilweise aus einer additionsvernetzenden Zwei-Komponenten-Silikon-Kautschuk-Masse (62) besteht.

13. Brustprothese (10) nach einem der vorhergehenden Ansprüche, **dadurch gekennzeichnet, dass** die Brustprothese (10) und/oder die Noppen wenigstens teilweise aus Schaumstoff ausgebildet ist bzw. sind.

## Claims

1. A breast prosthesis (10) having at least one contact surface (20) which is intended for the contact on a body surface, wherein the contact surface (20) has a plurality of moldings in the shape of nubs, **characterized in that** the nubs rise by different heights from the contact surface, wherein the height of the nubs reduces toward the rims of the contact surface and wherein air circulation and/or an exchange of air can be achieved between the contact surface (20) and the body surface by means of the nubs.

2. A breast prosthesis (10) in accordance with claim 1, **characterized in that** the nubs are formed in elliptic and/or round manner and/or in tear form.

3. A breast prosthesis (10) in accordance with one of the preceding claims, **characterized in that** the size of the nubs (30, 32, 34, 36, 130, 132, 134, 230, 232, 234) reduces at least toward one rim (80, 82, 84) and preferably in each case toward all rims (80, 82, 84), and is preferably the largest in the middle area of the contact surface (20).

4. A breast prosthesis (10) in accordance with one of the preceding claims, **characterized in that** at least one air passage (40) by means of which air can be led in and out is formed by the nubs in the state of the breast prosthesis applied on the body surface.

5. A breast prosthesis (10) in accordance with claim 4, **characterized in that** air can be led in and out through the air passage (40) by the chimney effect and/or by pump pressure or pressing in at least a part of the breast prosthesis (10).

6. A breast prosthesis (10) in accordance with one of the preceding claims, **characterized in that** the breast prosthesis (10) has at least one latent heat store (72) or at least partly comprises latent heat store material (72), with the latent heat store (72) or the latent heat store material (72) preferably being PCM material.

7. A breast prosthesis (10) in accordance with claim 6, **characterized in that** the latent heat store (72) or the latent heat store material (72) is arranged in the region of the contact surface (20).

8. A breast prosthesis (10) in accordance with either of claims 6 or 7, **characterized in that** the latent heat store (72) or the latent heat store material (72) is arranged in the region of the nubs.

9. A breast prosthesis (10) in accordance with one of the claims 6 to 8, **characterized in that** the nubs (30, 32, 34, 36, 130, 132, 134, 230, 232, 234), are formed at least partly by the latent heat store (72) or the latent heat store material (72) and/or at least partly comprise the latent heat store (72) or the latent heat store material (72).

10. A breast prosthesis (10) in accordance with one of the preceding claims, **characterized in that** the breast prosthesis (10) at least partly consists of an addition-curing two-component silicone rubber compound (62) or comprises an addition-curing two-component silicone rubber compound (62).

11. A breast prosthesis (10) in accordance with one of the preceding claims, **characterized in that** the breast prosthesis (10) is formed from at least one compound (62, 72) welded into plastic film (50) and/or simulates the shape of the breast, with the compound (62, 72) preferably at least partly comprising an addition-curing two-component silicone rubber compound (62); and/or **in that** the breast prosthesis (10) comprises a shell-shaped body of a soft elastically set plastic, preferably an addition-cured two-component silicone rubber compound (62) which is welded into plastic films (50) and thus forms a first chamber (60) and comprises a second chamber (70) facing the wearer during wear, adjoining the first chamber (60) and fillable with a filler material (72).

12. A breast prosthesis (10) in accordance with claim 11, **characterized in that** the nubs are formed by the compound (62, 72) welded into plastic film (50), with the compound (62, 72) preferably at least partly comprising an addition-curing two-component silicone rubber compound (62).

13. A breast prosthesis (10) in accordance with one of the preceding claims, **characterized in that** the breast prosthesis (10) and/or the nubs is/are made at least partly from foam.

## Revendications

1. Prothèse mammaire (10) comprenant au moins une surface d'appui (20), qui est destinée à l'appui sur une surface du corps,
la surface d'appui (20) comportant plusieurs protubérances sous la forme de bosses, **caractérisée en ce que** les bosses s'élèvent de la surface d'appui à différentes hauteurs, la hauteur des bosses diminuant vers les bords de la surface d'appui, et une circulation d'air et/ou un échange d'air entre la surface d'appui (20) et la surface du corps pouvant être obtenu(e) au moyen des bosses.

2. Prothèse mammaire (10) selon la revendication 1, **caractérisée en ce que** les bosses sont réalisées en forme d'ellipse et/ou rondes et/ou en forme de goutte.

3. Prothèse mammaire (10) selon l'une des revendications précédentes, **caractérisée en ce que** la taille des bosses (30, 32, 34, 36,130,132,134, 230, 232, 234) également diminue au moins vers un bord (80, 82, 84) et de préférence respectivement vers tous les bords (80, 82, 84), et est de préférence la plus grande dans la zone centrale de la surface d'appui (20).

4. Prothèse mammaire (10) selon l'une des revendications précédentes, **caractérisée en ce que** grâce aux bosses, dans l'état posé de la prothèse mammaire sur la surface du corps, au moins un canal d'air (40) est formé, au moyen duquel de l'air peut être amené et évacué.

5. Prothèse mammaire (10) selon la revendication 4, **caractérisée en ce que** de l'air peut être amené et évacué par le canal d'air (40) par effet de cheminée et/ou pression de pompage et/ou enfoncement d'au moins une partie de la prothèse mammaire (10).

6. Prothèse mammaire (10) selon l'une des revendications précédentes, **caractérisée en ce que** la prothèse mammaire (10) comporte au moins un régulateur thermique à changement d'état (72) ou est composée au moins en partie d'un matériau régulateur thermique à changement d'état (72), le régulateur thermique à changement d'état (72) ou le matériau régulateur thermique à changement d'état (72) étant de préférence un matériau MCP.

7. Prothèse mammaire (10) selon la revendication 6, **caractérisée en ce que** le régulateur thermique à changement d'état (72) ou le matériau régulateur thermique à changement d'état (72) est disposé dans la zone de la surface d'appui (20).

8. Prothèse mammaire (10) selon la revendication 6 ou 7, **caractérisée en ce que** le régulateur thermique à changement d'état (72) ou le matériau régulateur thermique à changement d'état (72) est disposé dans la zone des bosses.

9. Prothèse mammaire (10) selon l'une des revendications 6 à 8, **caractérisée en ce que** les bosses (30, 32, 34, 36, 130, 132, 134, 230, 232, 234) sont formées au moins en partie par le régulateur thermique à changement d'état (72) ou le matériau régulateur thermique à changement d'état (72) et/ou comprennent au moins en partie le régulateur thermique à changement d'état (72) ou le matériau régulateur thermique à changement d'état (72).

10. Prothèse mammaire (10) selon l'une des revendications précédentes, **caractérisée en ce que** la prothèse mammaire (10) est composée au moins en partie d'une masse à deux composants silicone/caoutchouc (62) réticulant par addition ou comprend une masse à deux composants silicone/caoutchouc (62) réticulant par addition.

11. Prothèse mammaire (10) selon l'une des revendications précédentes, **caractérisée en ce que** la prothèse mammaire (10) est formée au moins à partir d'une masse (62, 72) emballée sous vide dans du film en matière plastique (50) et/ou reproduit la forme du sein, la masse (62, 72) étant composée de préférence au moins en partie d'une masse à deux composants silicone/caoutchouc (62) réticulant par addition et/ou **en ce que** la prothèse mammaire (10) est composée d'un corps en forme de coque en une matière plastique à propriétés élastiques souples, de préférence une masse à deux composants silicone/caoutchouc (62) réticulée par addition, qui est emballé sous vide dans des films en matière plastique (50) et forme ainsi une première chambre (60), et d'une seconde chambre (70) pouvant être remplie avec une matière de remplissage (72), adjacente à la première chambre (60) et tournée vers la porteuse pendant le port.

12. Prothèse mammaire (10) selon la revendication 11, **caractérisée en ce que** les bosses sont formées par la masse (62, 72) emballée sous vide dans du film en matière plastique (50), la masse (62, 72) étant composée de préférence au moins en partie d'une masse à deux composants silicone/caoutchouc (62) réticulant par addition.

13. Prothèse mammaire (10) selon l'une des revendications précédentes, **caractérisée en ce que** la prothèse mammaire (10) et/ou les bosses est/sont formées au moins en partie en mousse.
